# EUROPÄISCHE PATENTSCHRIFT

(11) **EP 1 857 069 B1**
(45) Veröffentlichungstag und Bekanntmachung des Hinweises auf die Patenterteilung: **12.11.2008**
(21) Anmeldenummer: 06010122.7
(22) Anmeldetag: 16.05.2006
(51) Int. Cl.: A61B 19/00, A61G 13/12

(54) **Medizintechnische Beckenpositionierungs- und Trackingvorrichtung**
Medical hip positioning and tracking device
Dispositif médical de positionnement et de repérage de hanche

(43) Veröffentlichungstag der Anmeldung: 21.11.2007
(73) Patentinhaber: BrainLAB AG, 85551 Kirchheim/Heimstetten (DE)
(72) Erfinder: Tuma, Gregor, 81675 München (DE)
(74) Vertreter: Schwabe - Sandmair - Marx

(56) Entgegenhaltungen:
- EP-A- 1 522 300
- EP-A2- 1 481 635
- EP-A2- 1 570 782
- WO-A-02/100485
- DE-A1- 10 225 077
- US-A- 6 161 032
- US-A1- 2004 199 072
- US-A1- 2004 254 586

## Beschreibung

Die Erfindung betrifft eine medizintechnische Beckenpositionierungs- und Trackingvorrichtung. Bei der Durchführung medizinischer Behandlungen im Becken- bzw. Hüftbereich eines Patienten, beispielsweise beim Setzen von Implantaten, ist es von großem Vorteil wenn die Arbeiten mit Unterstützung durch ein medizintechnische Navigationssystem durchgeführt werden können. Bei dieser Art von bildunterstützter Chirurgie sollte das Becken des Patienten einerseits möglichst genau und bestenfalls unbeweglich positioniert und fixiert werden; andererseits sollte die Lage des Beckens zumindest anhand definierter Ebenen ermittelbar sein. Die Ermittlung von Positionsdaten für Köperteile oder Instrumente wird üblicherweise als "Tracking" bezeichnet, und sie liefert Positionseingabewerte für die medizintechnische Navigation.

Es gibt optische Trackingsysteme; eines ist beispielsweise aus der DE 196 39 615 A1 bekannt. Daneben sind elektromagnetische Trackingsysteme verfügbar, und solche Trackingsysteme werden beispielsweise in der EP 1 502 620 A1 und der US 6,332,089 B1 beschrieben. Elektromagnetische Trackingsysteme eignen sich sehr gut für solche Einsatzfälle, wo eine durchgehende Sichtlinie von einer Kamera zu einem Referenzmarker nicht immer aufrechterhalten werden kann, insbesondere wenn Patientenkörperteile getrackt werden sollen. Für gewöhnlich wird bei elektromagnetischen Trackingsystemen ein separat aufgestellter, externer Feldgenerator verwendet, um kleine Spulen im Magnetfeld zu orten und zu verfolgen, die an Patientenkörperteilen und/oder chirurgischen Instrumenten angebracht sind. Die Magnetfeldgeneratoren haben ein eingeschränktes Feldvolumen für Tracking-Zwecke, und sie müssen in einem sterilen Umfeld untergebracht werden. Es gibt auch Systeme, bei denen wegwerfbare Feldgeneratoren direkt am Patienten angebracht oder per Hand in die Nähe der Trackingspulen gehalten werden.

Eine Patientenpositionierungsvorrichtung mit einem Lokalisierungssystem für die Beckenebene, das auf einem optischen Tracking beruht, ist aus der US 2004/0254586 A1 bekannt.

US 2004/0199072 A1 offenbart eine Positionierungs- und Trackingvorrichtung mit mindestens einem Stützelement das an einer Patientenlagerung angeordnet ist, und mit einem Magnetfeldgenerator, wobei der Magnetfeldgenerator in dem Stützelement angeordnet ist.

Es ist die Aufgabe der vorliegenden Erfindung, eine medizintechnische Beckenpositionierungs- und Trackingvorrichtung bereitzustellen, welche optimale Bedingungen für den Einsatz eines Magnettrackingsystems liefert. Diese Aufgabe wird erfindungsgemäß durch eine Vorrichtung gemäß dem Anspruch 1 gelöst. Die Unteransprüche definieren bevorzugte Ausführungsformen der Erfindung.

Erfindungsgemäß wird eine medizintechnische Beckenpositionierungs- und Trackingvorrichtung bereitgestellt, die mindestens ein Beckenstützelement aufweist, das an einer Patientenlagerung angeordnet ist, sowie einen Magnetfeldgenerator zur Erzeugung eines Magnetfeldes für ein elektromagnetisches Trackingsystem, wobei der Magnetfeldgenerator in oder an dem bzw. einem Beckenstützelement angeordnet ist.

Die Erfindung stellt damit einen in allen Belangen optimierten Anordnungspunkt für den Magnetfeldgenerator zur Verfügung, weil das Beckenstützelement sich einerseits in unmittelbarer Nähe des Beckens befindet und so ein Feld erzeugt werden kann, das im Behandlungsbereich ausreichend stark, homogen und stabil ist.

Andererseits wird durch die erfindungsgemäße Anbringung des Magnetfeldgenerators verhindert, dass dieser oder seine Zuleitungen den Chirurgen bei der Arbeit im Wege sind. Mit anderen Worten gestattet die erfindungsgemäße Anordnung eine stationäre Positionierung des Magnetfeldgenerators in unmittelbarer Nähe der zu trackenden Instrumente oder Patientenkörperteile; der Magnetfeldgenerator wird sozusagen perfekt in das Behandlungs- bzw. Positionierungsumfeld integriert, und es ist nur eine einzige Vorrichtung notwendig, um ein elektromagnetisches Tracking durchzuführen und gleichzeitig das Becken zu fixieren. Das Beckenstützelement wird bei den meisten Beckenoperationen nämlich ohnehin verwendet.

Die erfindungsgemäße Vorrichtung ist einfach in der Verwendung und kann unsteril gehalten werden. Sie kann immer wieder verwendet werden. Die Nähe des Magnetfeldgenerators zum Operations- und Trackingbereich macht das Tracking selbst sehr genau, und dies innerhalb eines relativen kleinen Feldvolumens.

Bei einer bevorzugten Ausführungsform der vorliegenden Erfindung weist die Vorrichtung drei Beckenstützelemente auf, wobei ein Beckenstützelement den Magnetfeldgenerator aufweist und wobei an den anderen Beckenstützelementen Magnetspulen-Trackingeinrichtung angeordnet sind. Die Magnetspulen-Trackingeinrichtungen können grundsätzlich passive Resonanzspulen- oder aktive Magnetspulen-Trackingeinrichtungen mit eigener oder externer Energieversorgung und/oder Ansteuerung sein.

Bei einer vorteilhaften Ausgestaltung ist der Magnetfeldgenerator steril umschlossen in oder an dem bzw. einem Beckenstützelement angeordnet, insbesondere im Inneren des Beckenstützelements.

Die Beckenstützelemente können Stützelement-Halterungen aufweisen, an denen sie verstellbar angeordnet sind. Die Relativposition der drei Beckenstützelemente zueinander kann manuell verstellt werden, und sie wird durch das Tracking der Spulen in der Lage zum Ursprung (Magnetfeldgenerator) bekannt. Die Kenntnis der Einstellung bzw. der genauen Lage der Stützelemente zueinander ist z.B. wichtig für die weitere Referenzierung von Knochen oder Instrumenten zu dieser Lage. In diesem Fall ist es gemäß der Erfindung möglich, die Versorgungsleitungen für den Magnetfeldgenerator oder aktive Magnetspulen-Trackingeinrichtungen an oder in den Halterungen zu führen. Dies bringt den Vorteil mit sich, dass selbst Magnetspulen-Trackingeinrichtungen, die über Kabel mit Energie versorgt oder angesteuert werden, im Operationsgebiet so verlegt werden können, dass die Kabel den Chirurgen bzw. das chirurgische Team nicht bei der Arbeit stören.

Bei einer vorteilhaften Ausführungsvariante weist die erfindungsgemäße Vorrichtung zusätzliche Magnetspulen-Trackingeinrichtungen auf, die an Behandlungsgeräten oder behandlungsunterstützenden Geräten, insbesondere an Instrumenten, oder an Patientenkörperteilen, speziell am Becken, angeordnet werden können und welche sich innerhalb des Magnetfeldes befinden oder in dieses eingebracht werden können.

Eine Datenverarbeitungseinheit kann mit der erfindungsgemäßen Vorrichtung zur Verfügung gestellt werden, wobei diese Datenverarbeitungseinheit die Trackingdaten empfängt und verarbeitet. Die Datenverarbeitungseinheit ist bei einer vorteilhaften Ausführungsvariante ein Teil eines computergestützten medizintechnischen Navigationssystems. Die Trackingdaten können entweder durch die vorgenannte Ansteuerungsvorrichtung oder durch kabellose Datenübertragung an das Navigationssystem übermittelt werden.

Die erfindungsgemäße Vorrichtung kann gemäß einem weiteren Aspekt der Erfindung in Kombination mit einem optischen Trackingsystem bereitgestellt werden, welches optische Referenzmarkeranordnungen ortet und verfolgt, wobei die aus dem Magnettracking und dem optischen Tracking erhaltenen Positionsinformationen im Navigationssystem kombiniert oder gegenseitig ergänzt werden. Hierbei ist es wiederum von Vorteil, wenn mindestens eine der optischen Referenzmarkeranordnungen in einem vorbestimmten und/oder bekannten, insbesondere in einem festen Positionsverhältnis zu dem Magnetfeldgenerator und/oder zu einer der Magnetspulen-Trackingeinrichtungen angeordnet ist. Bei einem so ermöglichten simultanen Tracking über das elektromagnetische und das optische Trackingsystem gestattet die feste Relativpositionierung der Ursprünge beider Trackingsysteme eine Transformation auf das jeweilige andere Koordinatensystem, so dass es möglich wird, Instrumente, die mit optischen und/oder elektromagnetischen Trackingeinrichtungen versehen sind, simultan zu tracken.

Die Erfindung betrifft also eine Patientenpositionierungsvorrichtung, speziell zur Stabilisierung des Beckens während einer chirurgischen Behandlung, wobei die Patientenpositionierungsvorrichtung mit Magnettrackingeinheiten ausgestattet ist, sowie mit einem magnetischen Feldgenerator. Die Patientenpositionierungsvorrichtung ist ein integraler Teil eines chirurgischen Navigationssystems und kommuniziert ihre eigene Einstellung und Ausrichtung an den Navigationscomputer. Der magnetische Feldgenerator ist ein Teil der Tracking-Vorrichtung und kann die Beckenbewegung, chirurgische Instrumente und Relativpositionen von Fixierungseinrichtungen erfassen, welche das Becken an der Frontalebene fixieren.]

Die Erfindung wird im Weiteren anhand einer Ausführungsform näher erläutert. Sie kann alle hierin beschriebenen Merkmale einzeln sowie in jedweder sinnvollen Kombination umfassen. Die einzige beiliegende Figur zeigt eine erfindungsgemäße medizintechnische Beckenpositionierungs- und Trackingvorrichtung.

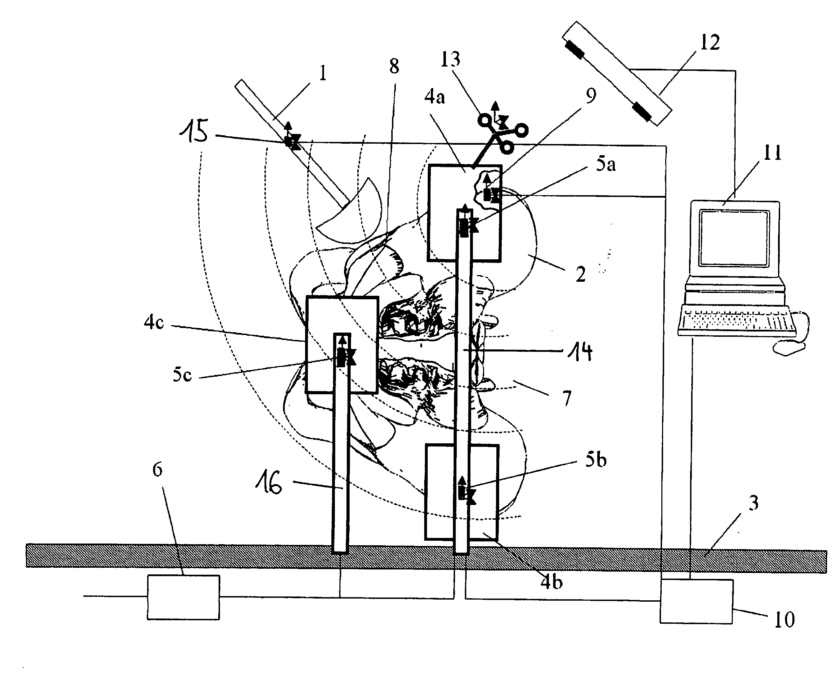

Das zentrale Element in der Zeichnung ist das Becken 2, das in einer Stellung gezeigt ist, bei der ein Patient seitlich auf einer Patientenliege 3 liegt.

Das Becken wird an seiner Vorderseite durch drei Beckenstützelemente 4a, 4b und 4c gehalten, von denen zwei (4a, 4b) an einer Stützelementhalterung 14 und ein weiteres (4c) an einer Stützelementhalterung 16 verschieblich angeordnet sind, so dass sie an die Beckenanatomie eines Patienten angepasst werden können. Die Stützelementhalterungen 14, 16 sind an der Patientenliege 3 befestigt, wobei es möglich, ist hier eine Verstellbarkeit entlang der Länge der Liege 3 vorzusehen.

Die Beckenstützelemente 4a, 4b und 4c sind in der Art von Haltekissen aufgebaut, die sich an drei Stellen des Beckens andrücken können. Die drei Stellen sind die beiden Schambeinhöcker, an denen das Stützelement 4c sitzt, sowie der linke und rechte vordere Darmbeinstachel, auf denen die beiden Stützelemente 4b und 4a zu liegen kommen. Im Beckenstützelement 4a ist der nur schematisch dargestellte Magnetfeldgenerator 5a untergebracht, der ein Magnettrackingfeld erzeugt, dessen Volumen mit dem Bezugszeichen 7 angedeutet ist. In den Beckenstützelementen 4b und 4c sind (auch hier nur schematisch dargestellt) Magnetspulen-Trackingeinrichtungen 5b, 5c angeordnet. Eine weitere Magnetspulen-Trackingeinrichtung 9 ist am Beckenknochen selbst angebracht, und auch das chirurgische Instrument 1 trägt eine Magnetspulen-Trackingeinrichtung 15. Zusammen mit dem Magnetfeldgenerator 5a bauen die Magnetspulen-Trackingeinrichtungen 5b, 5c, 1 und 9 ein Magnettrackingsystem auf.

Grundsätzlich können die Magnetspulen-Trackingeinrichtungen passive Resonanzspulen sein; es können aber auch aktive Magnetspulen-Trackingeinrichtungen zum Einsatz kommen. Im vorliegenden Fall sind die beiden Magnetspulen-Trackingeinrichtungen 5b und 5c solche, die mit Energie aus der Energieversorgung 6 versorgt werden und die entsprechenden Leitungen laufen durch die Halterungen 14 und 16. Auch der Magnetfeldgenerator 5a wird über die Energieversorgung 6 mit einer Leitung durch die Halterung 14 mit Energie versorgt. Die Becken-Trackingeinrichtung 9 sowie die Instrumenten-Trackingeinrichtung 15 sind entweder Resonanzspulen oder werden selbst und autark mit Energie versorgt (beispielsweise durch kleine Energieversorgungszellen).

Das Instrument 1 kann so frei im Magnetfeld 7 bewegt und beispielsweise zur Stelle 8 geführt werden, wo ein chirurgischer Eingriff am Becken 2 durchzuführen ist.

Ferner ist in der Figur eine Steuerungseinrichtung 10 zu sehen, welche einerseits den Magnetfeldgenerator 5a ansteuern kann und andererseits die Positionsdaten der Magnetspulen-Trackingeinrichtungen an ein medizintechnisches Navigationssystem 11 weiterleitet. Hierzu sind verschiedene Verbindungslinien dargestellt, die von den Magnetspulen-Trackingeinrichtungen zur Steuerungseinheit 10 und von dort wieder zum Navigationssystem 11 führen.

Die gesamte Vorrichtung umfasst noch ein optisches Trackingsystem 12, das über eine optische Referenzmarkeranordnung 13 eine Postionsortung für das Beckenstützelement 4a durchführen kann, das in einem festen Positionsverhältnis zum Magnetfeldgenerator 5a steht.

Die Vorrichtung wird dazu verwendet, das chirurgische Instrument 1 elektromagnetisch bzgl. seiner relativen Position zum Becken 2 zu tracken um eine positionell definierte Behandlungsvorbereitung zu erzielen (wenn das Instrument 1 ein Behandlungsvorbereitungsinstrument ist) oder auch eine korrekte Ausrichtung eines Implantats (wenn das Instrument ein Implantathalter ist). Der Patient liegt auf der Patientenliege 3 und sein Becken 2 ist in der erfindungsgemäßen Vorrichtung fixiert, und zwar auf der dargestellten Vorderseite über die drei Beckenstützelemente (Stabilisatoren) 4a, 4b und 4c. Die dreidimensionale Position der Magnetspulen-Trackingeinrichtungen 5b, 5c gegenüber dem Ursprung bei dem Generator 5a wird gemäß dem aktuell eingestellten Zustand der Beckenpositionierungsvorrichtung berechnet, wobei das vom Generator 5a erzeugte Feld 7 groß bzw. stark genug ist, um sowohl die beiden Trackingeinrichtungen 5b und 5c als auch die Trackingeinrichtungen 1 und 9 am Instrument bzw. am Becken zu tracken. Dabei repräsentieren die drei Koordinaten, die durch die Trackingeinrichtungen 5b und 5c sowie durch den Magnetfeldgenerator 5a definiert werden die vordere Beckenebene im Raum als Referenz für die Ausrichtung des Instruments. 1. Bewegungen des Beckens 2 gegenüber dem Instrument 1 und/oder der Positionierungsvorrichtung können durch die Magnetspulen-Trackingeinrichtung 9 verfolgt werden, die am Beckenknochen angeordnet ist.

Als Materialien für die Beckenstützelemente und ihre Halterungen sollten solche ausgewählt werden, die das Magnetfeld nicht stören.

Mit Hilfe der Positionsdaten für die Trackingeinrichtungen kann das Navigationssystem 11 dem Benutzer eine Bildunterstützung zur Verfügung stellen. Dabei können die Positionsdaten entweder über die Steuerungseinheit 10 oder per drahtloser Übertragung an das Navigationssystem übermittelt werden.

Eine zusätzliche Navigationsunterstützung bietet das optische Kameratrackingsystem 12, das ebenfalls mit dem Navigationssystem 11 in Verbindung steht. Es umfasst eine Referenzanordnung 13 aus drei Referenzmarkern, die entweder gegenüber dem magnetischen Feldgenerator 5a und/oder gegenüber einer der Trackingeinrichtungen angeordnet ist, so dass die Magnettrackingeinrichtung insgesamt simultan sowohl elektromagnetisch als auch optisch getrackt wird. Diese Positionierung der Ursprünge der beiden Trackingsysteme gestattet dann eine Transformation in das jeweils andere Koordinatensystem, um zusätzlich eine simultane Ortung und Verfolgung von Instrumenten zu gestatten, die mit optischen und/oder elektromagnetischen Trackingeinrichtungen versehen sind.

Es wird somit durch die vorliegende Erfindung ein optimiertes elektromagnetisches Tracking bereitgestellt, bei dem durch die geeignete Anordnung des Feldgenerators ein stabiles, sicheres und genaues elektromagnetisches Tracking gesichert wird, das noch in einfacher Weise durch ein optisches Trackingsystem ergänzt werden kann.

## Patentansprüche

1. Medizintechnische Beckenpositionierungs- und Trackingvorrichtung mit mindestens zwei Beckenstützelementen (4a, 4b, 4c), die an einer Patientenlagerung (3) angeordnet sind, und mit einem Magnetfeldgenerator (5a) zur Erzeugung eines Magnetfeldes für ein elektromagnetisches Trackingsystem, wobei Magnetfeldgenerator (5a) in oder an einem Beckenstützelement (4a) angeordnet ist, und wobei an dem oder den anderen Beckenstützelementen (4b, 4c) Magnetspulen-Trackingeinrichtungen (5b, 5c) angeordnet sind.

2. Vorrichtung nach Anspruch 1, **dadurch gekennzeichnet, dass** sie drei Beckenstützelemente (4a, 4b, 4c) aufweist.

3. Vorrichtung nach Anspruch 1 oder 2, **dadurch gekennzeichnet, dass** die Magnetspulen-Trackingeinrichtungen passive Resonanzspulen oder aktive Magnetspulen-Trackingeinrichtungen (5b, 5c) mit eigener oder externer Energieversorgung (6) und/oder Ansteuerung (10) sind.

4. Vorrichtung nach einem der Ansprüche 1 bis 3, **dadurch gekennzeichnet, dass** der Magnetfeldgenerator (5a) steril umschlossen in oder an dem bzw. einem Beckenstützelement (4a, 4b, 4c) angeordnet ist, insbesondere im Inneren des Beckenstützelements (4a).

5. Vorrichtung nach einem der Ansprüche 1 bis 4, **dadurch gekennzeichnet, dass** das bzw. die Beckenstützelement(e) (4a, 4b, 4c) Stützelement-Halterungen (14) aufweise, an denen sie verstellbar angeordnet sind, wobei insbesondere die Versorgungsleitungen für den Magnetfeldgenerator (5a) oder aktive Magnetspulen-Trackingeinrichtungen (5b, 5c) an oder in den Halterungen (14) geführt werden.

6. Vorrichtung nach Anspruch 5, bei der die Relativposition der Beckenelemente zueinander verstellt werden kann und durch das Tracking der Magnetspulen-Trackingeinrichtungen (5b, 5c) in der Lage zum Ursprung am Magnetfeldgenerator (5a) ermittelt werden kann.

7. Vorrichtung nach einem der Ansprüche 1 bis 6, **dadurch gekennzeichnet, dass** sie zusätzliche Magnetspulen-Trackingeinrichtungen (9, 15) umfasst, die an Behandlungsgeräten oder behandlungsunterstützenden Geräten, insbesondere an Instrumenten (1), oder an Patientenkörperteilen, insbesondere am Becken (2), angeordnet werden können, welche sich innerhalb des Magnetfeldes befinden oder in dieses eingebracht werden können.

8. Vorrichtung nach einem der Ansprüche 1 bis 7, **dadurch gekennzeichnet, dass** sie eine Datenverarbeitungseinheit aufweist, welche die Trackingdaten empfängt und verarbeitet.

9. Vorrichtung nach Anspruch 8, **dadurch gekennzeichnet, dass** die Datenverarbeitungseinheit ein Teil eines computergestützten medizintechnischen Navigationssystems (11) ist.

10. Vorrichtung nach Anspruch 9, **dadurch gekennzeichnet, dass** sie in Kombination mit einem optischen Trackingsystem (12) bereitgestellt wird, welches optische Referenzmarkeranordnungen (13) ortet und verfolgt, wobei die aus dem Magnettracking und dem optischen Tracking erhaltenen Positionsinformationen im Navigationssystem (11) kombiniert oder gegenseitig ergänzt werden.

11. Vorrichtung nach Anspruch 10, **dadurch gekennzeichnet, dass** mindestens eine der optischen Referenzmarkeranordnungen (13) in einem vorbestimmten und/oder bekannten, insbesondere in einem festen Positionsverhältnis zu dem Magnetfeldgenerator (5a) und/oder zu einer der Magnetspulen-Trackingeinrichtungen (5b, 5c) angeordnet ist.

## Claims

1. A medical pelvic positioning and tracking device, comprising at least two pelvic support elements (4a, 4b, 4c) arranged on a patient placement (3), and comprising a magnetic field generator (5a) for generating a magnetic field for an electromagnetic tracking system, wherein the magnetic field generator (5a) is arranged in or on a pelvic support element (4a), and wherein magnetic coil tracking means (5b, 5c) are arranged on the other pelvic support element or elements (4b, 4c).

2. The device according to claim 1, **characterised in that** it comprises three pelvic support elements (4a, 4b, 4c).

3. The device according to claim 1 or 2, **characterised in that** the magnetic coil tracking means are passive resonance coils or active magnetic coil tracking means (5b, 5c) with an integrated or external power supply (6) and/or control unit (10).

4. The device according to any one of claims 1 to 3, **characterised in that** the magnetic field generator (5a) is arranged, sterilely enclosed, in or on the pelvic support element or a pelvic support element (4a, 4b, 4c), in particular in the interior of the pelvic support element (4a).

5. The device according to any one of claims 1 to 4, **characterised in that** the pelvic support element(s) (4a, 4b, 4c) comprise support element holders (14) on which they are adjustably arranged, wherein in particular the supply lines for the magnetic field generator (5a) or active magnetic coil tracking means (5b, 5c) are guided on or in the holders (14).

6. The device according to claim 5, **characterised in that** the position of the pelvic elements relative to each other can be adjusted, and their position relative to the origin on the magnetic field generator (5a) can be ascertained by tracking the magnetic coil tracking means (5b, 5c).

7. The device according to any one of claims 1 to 6, **characterised in that** it additionally comprises magnetic coil tracking means (9, 15) which can be arranged on treatment apparatus or treatment-assisting apparatus, in particular on instruments (1), or on parts of the patient's body, in particular on the pelvis (2), and which are situated within the magnetic field or can be introduced into it.

8. The device according to any one of claims 1 to 7, **characterised in that** it comprises a data processing unit which receives and processes the tracking data.

9. The device according to claim 8, **characterised in that** the data processing unit is part of a computer-assisted medical navigation system (11).

10. The device according to claim 9, **characterised in that** it is provided in combination with an optical tracking system (12) which localises and tracks optical reference marker arrays (13), wherein the positional information obtained from magnetic tracking and the positional information obtained from optical tracking is combined or mutually supplemented in the navigation system (11).

11. The device according to claim 10, **characterised in that** at least one of the optical reference marker arrays (13) is arranged in a predetermined and/or known, in particular fixed, positional relationship with respect to the magnetic field generator (5a) and/or one of the magnetic coil tracking means (5b, 5c).

## Revendications

1. Dispositif médical de positionnement et de localisation du bassin, comportant au moins deux éléments d'appui de bassin (4a, 4b, 4c) qui sont disposés sur un support de patient (3), et comportant un générateur de champ magnétique (5a) pour produire un champ magnétique pour un système électromagnétique de localisation, le générateur de champ magnétique (5a) étant disposé dans ou sur un élément d'appui de bassin (4a), et des dispositifs de localisation à bobines magnétiques (5b, 5c) étant disposés sur les autres éléments d'appui de bassin (4b, 4c).

2. Dispositif selon la revendication 1, **caractérisé en ce qu'**il comporte trois éléments d'appui de bassin (4a, 4b, 4c).

3. Dispositif selon la revendication 1 ou 2, **caractérisé en ce que** les dispositifs de localisation à bobines magnétiques sont des bobines passives à résonance ou des dispositifs actifs de localisation à bobines magnétiques (5b, 5c) avec alimentation en énergie (6) et/ou commande (10) qui leur sont propres ou externes.

4. Dispositif selon l'une quelconque des revendications 1 à 3, **caractérisé en ce que** le générateur de champ magnétique (5a) est enfermé de manière stérile ou est disposé dans ou sur le ou un élément d'appui de bassin (4a, 4b, 4c), en particulier à l'intérieur de l'élément d'appui de bassin (4a).

5. Dispositif selon l'une quelconque des revendications 1 à 4, **caractérisé en ce que** le ou les élément(s) d'appui de bassin (4a, 4b, 4c) comporte(nt) des éléments d'appui (14) sur lesquels ils sont disposés réglables, les lignes d'alimentation pour le générateur de champ magnétique (5a) ou des dispositifs actifs de localisation à bobines magnétiques (5b, 5c) étant en particulier guidées sur ou dans les supports (14).

6. Dispositif selon la revendication 5, dans lequel les positions relatives des éléments de bassin peuvent être réglées les unes par rapport aux autres et peuvent être déterminées par la localisation des dispositifs de localisation à bobines magnétiques (5b, 5c) dans leur position par rapport à l'origine, sur le générateur de champ magnétique (5a).

7. Dispositif selon l'une quelconque des revendications 1 à 6, **caractérisé en ce qu'**il comprend des dispositifs supplémentaires de localisation à bobines magnétiques (9, 15) qui peuvent être disposés sur des appareils de traitement ou des appareils d'assistance au traitement, en particulier sur des instruments (1), ou sur des parties corporelles d'un patient, en particulier sur le bassin (2), lesquels se trouvent à l'intérieur du champ magnétique ou peuvent être introduits dans celui-ci.

8. Dispositif selon l'une quelconque des revendications 1 à 7, **caractérisé en ce qu'**il comporte une unité de traitement de données qui reçoit et traite les données de localisation.

9. Dispositif selon la revendication 8, **caractérisé en ce que** l'unité de traitement de données fait partie d'un système médical de navigation (11) assisté par ordinateur.

10. Dispositif selon la revendication 9, **caractérisé en ce qu'**il est fourni en combinaison avec un système optique de localisation (12) qui localise et suit des agencements de repères optiques de référence (13), les informations de position, obtenues à partir de la localisation magnétique et de la localisation optique, étant combinées ou complétées réciproquement dans le système de navigation (11).

11. Dispositif selon la revendication 10, **caractérisé en ce qu'**au moins l'un des agencements de repères optiques de référence (13) est disposé dans un rapport de position prédéterminé et/ou connu, en particulier fixe, par rapport au générateur de champ magnétique (5a) et/ou par rapport à l'un des dispositifs de localisation à bobines magnétiques (5b, 5c).
